# EUROPEAN PATENT APPLICATION

(11) **EP 1 925 293 A2**
(43) Date of publication of application: **28.05.2008**
(21) Application number: 08004692.3
(22) Date of filing: 18.10.2004
(51) Int. Cl.: A61K 9/12, A61P 11/06

(54) **Pharmaceutical aerosol compositions**

(30) Priority: 20.10.2003 US 512725
(62) Divisional of application: 04795506.7
(71) Applicant: Schering Corporation, Kenilworth, NJ 07033-0530 (US)
(72) Inventor: Berry, Julianne, Westfield NJ 07090 (US); Sherwood, Jill K., Edison NJ 08820 (US); Chaudhry, Saeed, Clifton NJ 07011 (US); Sequeira, Joel A., Edison NJ 08820 (US)
(74) Representative: UEXKÜLL & STOLBERG

(57) **Abstract**

Disclosed is a method for manufacturing metered dose inhalers having a metering valve to deliver a dose containing an aerosol suspension formulation, said aerosol suspension formulation comprising: an effective amount of Mometasone Furoate, Formoterol or a combination thereof; a suspension medium selected from the group consisting of 1,1,1,2,3,3,3,-heptafluoropropane, 1,1,1,2-tetrafluoroethane; and a solvent that is ethanol; said method comprising that the level of non-volatile residue contained in said formulation is determined and controlled to be less than about 500 µg.

## Description

### CROSS REFERENCE TO RELATED APPLICATION

This application claims benefit of priority to U.S. Provisional Patent Application Serial No. 60/512,725, the entirety of which is hereby incorporated by reference.

### BACKGROUND OF THE INVENTION

This invention relates to the improved formulations for the treatment of corticosteroid and β agonist responsive diseases of the upper and lower airway passages and lungs, such as allergic rhinitis and asthma, by orally or intranasally administering to those passages and lungs an amount of a corticosteroid, a β agonist or a combination thereof, effective for treating such diseases.

A metered dose inhaler is the most commonly used device for delivering drugs to the respiratory tract in the treatment of pulmonary diseases such as asthma. The MDI device generally comprises the formulation, a metering valve, a container and actuator. Medicaments are delivered to the patient as an aerosol of fine droplets by the atomization of the liquid phase of the formulation. The driving force for atomization is provided by the evaporation of the propellant within the actuator nozzle.

The aerodynamic particle size distribution (PSD) of the product is an important parameter that needs to be carefully controlled since it determines where the aerosol will deposit in the respiratory tract and is closely linked to the efficacy of the delivered medication. Aerosol droplets that are less than or equal to 5 µm in diameter are considered respirable and have the highest probability of reaching the lower respiratory tract. Medications used for local treatment of the lung generally target the size range of 2-5 µm.

MDI's have components that come into contact with the liquid formulations such as the interior of the canister and the metering valve. Any physical or chemical interaction of the packaging material with the formulation may impact the performance of the product, e.g., reduce the PSD of the active ingredient upon delivery of the medication to the lungs. For instance, if contact of the liquid formulation with the valve components is significant, such as the case when the canister is stored in the valve down orientation, there is increased potential for materials from the valve components to dissolve or leach into the product. These leachable materials, such as Plastanox 2246, dehyrodoabietic acid, Irganox 245 and Irganox 259, which are non-volatile materials, could then contribute to an increase in the mass median aerodynamic diameter (MMAD) and the corresponding decrease in fine particle dose (FPD) of the product by interacting with the drug and/or reducing the spray evaporation rate.

Accordingly, there exists a need for formulations for the treatment of asthma and allergies, that do not suffer from the aforementioned infirmities with regards to leachable materials.

### SUMMARY OF THE INVENTION

Accordingly, there is disclosed a metered dose inhaler having a metering valve to deliver a dose containing an aerosol suspension formulation, said aerosol suspension formulation comprising: an effective amount of Mometasone Furoate, Formoterol or a combination thereof; a suspension medium selected from the group consisting of 1,1,1,2,3,3,3,-heptafluoropropane, 1,1,1,2-tetrafluoroethane; and a solvent that is ethanol; wherein said formulation contains less than about 500 µg of non-volatile residue as measured by ultraviolet spectroscopy.

### WRITTEN DESCRIPTION OF THE INVENTION

Mometasone Furoate, the active component of Nasonex® is an antiinflammatory corticosteroid having the chemical name, 9, 21-Dichloro-11(beta), 17-dihydroxy-16(alpha)-methylpregna-1, 4-diene-3, 20-dione 17-(2 Furoate). This component may be present in an amount of about 25 to about 500 micrograms per actuation of the MDI. This product is available from Schering-Plough Corporation, Kenilworth, New Jersey.

Formoterol Fumarate is a selective beta 2-adrenergic bronchodilator. Its chemical name is (±)-2-hydroxy-5-[(1RS)-1-hydroxy-2-[[(1RS)-2-(4-methoxyphenyl)-1-methylethyl]- amino]ethyl]formanilide Fumarate dihydrate. This component may be present in an amount of about 3 to about 50 micrograms per actuation. This product is available commercially from Novartis Corporation, East Hanover, New Jersey and Schering-Plough Corporation, Kenilworth, New Jersey under the trademark Foradil®.

Propellant-based pharmaceutical aerosol formulations in the art typically use a mixture of liquid chlorofluorocarbons as the propellant, although many others use a single propellant. As is known in the art, the propellant serves as a vehicle for both the active ingredients and excipients. Fluorotrichloromethane, dichlorodifluoromethane and dichlorotetrafluoroethane are the most commonly used propellants in aerosol formulations for administration by inhalation. Such chlorofluorocarbons (CFCs), however, have been implicated in the destruction of the ozone layer and their production is being phased out. Non-CFC propellants are said to be less harmful to the ozone than many chlorofluorocarbon propellants. Non-CFC propellants systems must meet several criteria for pressurized metered dose inhalers. They must be non-toxic, stable and non-reactive with the medicament and the other major components in the valve/actuator. One propellant that has been found to be suitable is CF₃CHFCF₃, also known as HFA 227, HFC 227 or 1,1,1,2,3,3,3 heptafluoropropane. Another such propellant for use in metered dose inhalers is CF₃CH₂F, also known as 1,1,1,2-tetrafluoroethane or HFA 134a. Both are considered to be within the scope of the present invention.

The processes for producing the formulations of the present invention preferably utilize HFA 227 or HFA 134a, or a combination thereof, in combination with Mometasone Furoate and optionally, Formoterol Fumarate, a liquid excipient, and a surfactant. The excipient facilitates the compatibility of the medicament with the propellant and also lowers the discharge pressure to an acceptable range, i.e., about 2.76-5.52 X 10⁵ newton/meter² absolute (40 to 80 psi), preferably 3.45-4.83 X 10⁵ newton/meter² absolute (50 to 70 psi). The excipient chosen must be non-reactive with the medicaments, relatively non-toxic, and should have a vapor pressure below about 3.45 X 10⁵ newton/meter² absolute (50 psi).

As used hereinafter the term "medium chain fatty acids" refers to chains of alkyl groups terminating in a -COOH group and having 6-12 carbon atoms, preferably 8-10 carbon atoms. The term "short chain fatty acids" refers to chains of alkyl groups terminating in a -COOH group and having 4-8 carbon atoms. The term "alcohol" includes C₁-C₃ alcohols, such as methanol, ethanol and isopropanol.

Among the preferred excipients are: propylene glycol diesters of medium chain fatty acids available under the tradename Miglyol 840 (from Huls America, Inc. Piscataway, N.J.); triglyceride esters of medium chain fatty adds available under the tradename Miglyol 812 (from Huls); perfluorodimethylcyclobutane available under the tradename Vertrel 245 (from E. I. DuPont de Nemours and Co. Inc. Wilmington, Del.); perfluorocyclobutane available under the tradename octafluorocyclobutane (from PCR Gainsville, Fla.); polyethylene glycol available under the tradename EG 400 (from BASF Parsippany, N.J.); menthol (from Pluess-Stauffer International Stanford, Conn.); propylene glycol monolaurate available under the tradename lauroglycol (from Gattefosse Elmsford, N.Y.); diethylene glycol monoethylether available under the tradename Transcutol (from Gattefosse); polyglycolized glyceride of medium chain fatty adds available under the tradename Labrafac Hydro WL 1219 (from Gattefosse); alcohols, such as ethanol, methanol and isopropanol; eucalyptus oil available (from Pluses-Stauffer International); and mixtures thereof.

A surfactant is frequently included in aerosol formulations, for purposes such as assisting with maintaining a stable suspension of the drug and also lubricating the metering valve. The formulation of the present invention does not require a surfactant for maintenance of ready dispersability (such as by moderate agitation immediately prior to use), as the drugs form loose floccules in the propellant and does not exhibit a tendency to settle or compact. In the case of HFA 227 upon undisturbed storage, the drug particles remain suspended in their flocculated state. Thus, a surfactant optionally may be added to lower the surface and interfacial tension between the medicaments and the propellant. Where the medicaments, propellant and excipient are to form a suspension, a surfactant may or may not be required. Where the medicament, propellant and excipient are to form a solution, a surfactant may or may not be necessary, depending in part, on the solubility of the particular medicament and excipient.

The surfactant may be any suitable, non-toxic compound that is non-reactive with the medicament and that substantially reduces the surface tension between the medicament, the excipient and the propellant and/or acts as a valve lubricant. Among the preferred surfactants are: oleic acid available under the tradenames Mednique 6322 and Emersol 6321 (from Cognis Corp., Cincinnati, Ohio); cetylpyridinium chloride (from Arrow Chemical, Inc. Westwood, N.J.); soya lecithin avaitable under the tradename Epikuron 200 (from Lucas Meyer Decatur, III.); polyoxyethylene(20) sorbitan monolaurate available under the tradename Tween 20 (from ICI Specialty Chemicals, Wilmington, Del.); polyoxyethylene(20) sorbitan monostearate available under the tradename Tween 60 (from ICI); polyoxyethylene(20) sorbitan monooleate available under the tradename Tween 80 (from ICI); polyoxyethylene (10) stearyl ether available under the tradename Brij 76 (from ICI); polyoxyethylene (2) oleyl ether available under the tradename Brij 92 (frown ICI); Polyoxyethylene-polyoxypropylene-ethylenediamine block copolymer available under the tradename Tetronic 150 R1 (from BASF); polyoxypropylene-polyoxyethylene block copolymers available under the tradenames Pluronic L-92, Pluronic L-121 end Pluronic F 68 (from BASF); castor oil ethoxylate available under the tradename Alkasurf CO-40 (from Rhone-Poulenc Mississauga Ontario, Canada); and mixtures thereof.

A certain minimum level of ethanol is preferred to provide consistent and predictable delivery of the drug from a metered dose dispenser. This minimum level is about 1 weight percent of the total formulation, that results in a marginally acceptable drug delivery. Increased amounts of ethanol generally improve drug delivery characteristics. However, to prevent drug crystal growth in the formulation, it is preferred to limit the concentration of ethanol. Experimental data indicate that the ratio of the weight of Mometasone Furoate to the weight of ethanol is important in preventing particle size increases.

This invention further relates to the improvement in the quality from both a stability and performance perspective of the Mometasone Furoate for use in either oral and nasal MDI suspensions. The Mometasone may be used either alone or in combination with other drug substances, such as, for instance, Formoterol. The improved formulations relate specifically with regards to using a valve with low non-volatile residue (hereinafter "NVR"). For Mometasone Furoate MDI, the quality of the drug product is linked to the amount of NVR in the valve components.

Prolonged contact of the Mometasone Furoate MDI product with a valve containing materials with high levels of leachables and/or lubricants, i.e. silicone oil, resulted in an unacceptable decrease in the % fine particles (% FP) produced in the emitted aerosol spray. In fact, a direct correlation was found between the level of these NVR materials in the product and the reduction in % fine particles.

The correlation of NVR to %FP reduction was observed when the NVR was expressed in the following ways:
1) Total NVR (determined gravimetrically)
2) NVR detected by UV/Vis spectroscopy
3) Valve extractables (Potential NVR materials)
4) Valve lubricant levels
Examples of the impact of NVR on %FP are given below:

### Example 1

The impact of temperature on the total NVR (determined gravimetically) and %FP for samples stored at a temperature range of from 25°C vs. 40°C for 6 months in the inverted orientation was determined to yield the following results:

| Temperature | Total NVR | Reduction in %FP |
|---|---|---|
| 25°C | 1.73 mg | < 5% |
| 40°C | 3.31 mg | >10% |

### Example 2

Impact of orientation on Ultraviolet/Visible ("UV/V") light is detectable NVR (as measured by HPLC) and %FP after storage at 6 months at 40°C and 75% relative humidity.

| Orientation | UV/V NVR | %FP |
|---|---|---|
| Inverted | 550 µg | 45 |
| Upright | 360 µg | 55 |

### Example 3

Impact of valve elastomers on extractables and %FP after storage at 6 months in inverted orientation and at 40°C and 75% relative humidity. The extractables were determined on the valve prior to product contact and as detected by HPLC with UV/Vis, detection.

| Elastomer | Extractables | Reduction in %FP |
|---|---|---|
| Neoprene | 6 mg/valve | >10% |
| EPDM | << 6 mg/valve | <5% |

### Example 4

Test were conducted and yielded the following results.

| **Config** | **Silicone** | **Initial** | | **1 Month RH4** | | **3 Months RH4** | | **6 Months RH4** | |
|---|---|---|---|---|---|---|---|---|---|
| | **(µg)** | **%FP** | **MMAD** | %Δ | **MMAD** | %Δ | **MMAD** | %Δ | **MMAD** |
| | | | | | | *-3%* | *2.5* | | |
| 35 | 0 | 75 | 2.5 | -5% | 2.8 | -7% | 2.7 | -5% | 2.8 |
| | | | | | | -5% | 2.6 | -6% | 2.6 |
| 34 | <50 | 77 | 2.5 | -10% | 2.7. | -13% | 2.8 | -13% | 2.8 |
| | | | | | | | | | |
| 31 | 100 | 76 | 2.5 | -13% | 2.8 | ND | ND | ND | ND |
| | | | | | | *-9%* | *2.7* | | |
| 32 | 300 | 78 | 2.5 | -14% | 2.9 | -17% | 2.8 | -19% | 3.0 |
| | | | | | | | | | |
| Inverted except where in italics | | | | | | ND= No data | | | |
| n=2 in blue, otherwise n=3 | | | | | | MMAD in µm | | | |

As is apparent, as the silicone levels increased there was a significant change in the decrease of percent fine particles. In addition, inverted samples that were in constant contact with the valve showed more change in %FP than upright samples.

### Example 5

Impact of valve lubricant (silicone oil) levels on %FP (initial storage). The extractables were determined on the valve prior to product contact and as detected by UVN as measured by HPLC.

| Silicone Level | %FP |
|---|---|
| » 50 µg/valve | 41% |
| < 50 mg/valve | 57% |

Not only did the product containing low NVR demonstrate a higher percentage Fine Particle and improved particle size stability (i.e., less change in %FP over time), but the product also displayed improved dose retention in the metering chamber and dose content uniformity as shown in the following examples.

### Example 6

Mometasone Furoate MDI product with a valve containing high NVR (high silicone level and/or high extractable level) had shown substantial leakage of the dose from the metering chamber after placement of the product in the valve up orientation (i.e., loss of prime; "LOP"). This resulted in the failure of the product to meet the requirements for patient use testing. On the other hand, when the Mometasone Furoate MDI product was comprised of a valve with low NVR (low silicone level and/or low extractable level), the dose was retained in the metering chamber and the product passed the requirements for patient use testing. As will be appreciated by one of skill. it is preferable if the valve contains a pre-extracted gasket in the neck of the valve.

### Example 7

The drug dose uniformity (DDU) of this product can be affected by the valve material as shown by a substantial drop in the % label claim (% LC) using a high silicone level and/or high extractable level valves after storage valve down at 40°C. At the same time, the % LC (as obtained initially) was maintained for the product when it contained low silicone level and/or low extractable level in the valves. In the case of the Mometasone Furoate MDI, the following NVR levels have shown to produce an acceptable product:
Total NVR (determined gravimetrically) < 3 mg/can
NVR detected by UV/Vis spectroscopy < 500 µg/can
Valve extractables (Potential NVR materials) < 6 mg/valve
Valve lubricant level <50 µg/valve
At levels higher than above, the product has at times been found to be unacceptable.

The foregoing descriptions of various embodiments of the invention are representative of various aspects of the invention, and are not intended to be exhaustive or limiting to the precise forms disclosed. Many modifications and variations undoubtedly will occur to those having skill in the art. It is intended that the scope of the invention shall be fully defined solely by the appended claims.

Further embodiments of the invention are described in the subsequent paragraphs A to L.
A. A metered dose inhaler having a metering valve to deliver a dose containing an aerosol suspension formulation, said aerosol suspension formulation comprising: an effective amount of a compound selected from the group consisting of Mometasone Furoate, Mometasone Furoate Monohydrate, Formoterol, Formoterol Fumarate and/or a combination of any one of the same; a suspension medium selected from the group consisting of 1,1,1,2,3,3,3,-heptafluoropropane, 1,1,1,2-tetrafluoroethane; and a solvent that is ethanol; wherein said formulation contains less than about 500 µg of non-volatile residue as measures by ultraviolet spectroscopy.
B. A metered dose inhaler as described in paragraph A, wherein the delivered dose upon actuation of the metered dose inhaler contains the compound Mometasone Furoate in an amount of about 50 µg to about 400 µg.
C. A metered dose inhaler as described in paragraph A, wherein the median particle size of the Mometasone Furoate that is delivered upon actuation of said metered dose inhaler remains less than about 5 microns.
D. A metered dose inhaler as described in paragraph A, wherein the formulation has less than about 3 mg of non-volatile residue by weight per formulation.
E. A metered dose inhaler as described in paragraph A, wherein the formulation has less than about 6 mg of valve extractables in the metering valve.
F. A metered dose inhaler as described in paragraph A, further comprising a lubricant, wherein the formulation has less than about 100 µg of lubricant in the valve.
G. A metered dose inhaler as described in paragraph F, wherein the formulation has less than about 50 µg of lubricant in the valve.
H. A metered dose inhaler as described in paragraph A, wherein the delivered dose upon actuation of the metered dose inhaler contains the compound Formoterol in an amount of about 6 µg to about 12 µg.
I. A metered dose inhaler as described in paragraph H, wherein the particle size of the Formoterol that is delivered upon actuation of said metered dose inhaler remains less than about 5 microns.
J. A metered dose inhaler as described in paragraph A, further comprising a surfactant.
K. A metered dose inhaler as described in paragraph A, wherein the delivered dose upon actuation of the metered dose inhaler contains the compound Mometasone Furoate in an amount of about 50 µg to about 400 µg and wherein the delivered dose upon actuation of the metered dose inhaler contains the compound Formoterol in an amount of about 6 µg to about 12 µg.
L. A metered dose inhaler as described in paragraph K, wherein the particle size of the Formoterol and the Mometasone Furoate that is delivered upon actuation of said metered dose inhaler remains less than about 5 microns.

## Claims

1. A method of manufacturing a metered dose inhaler having a metering valve to deliver a dose containing an aerosol suspension formulation, said aerosol suspension formulation comprising: an effective amount of a compound selected from the group consisting of Mometasone Furoate, Mometasone Furoate Monohydrate, Formoterol, Formoterol Fumarate and/or a combination of any one of the same; a suspension medium selected from the group consisting of 1,1,1,2,3,3,3,-heptafluoropropane, 1,1,1,2-tetrafluoroethane; and a solvent that is ethanol; said method comprising that the level of non-volatile residue contained in said formulation is determined and controlled to be less than about 500 µg.

2. The method according to claim 1, wherein the level of non-volatile residue is determined by means of ultraviolet spectroscopy.

3. The method according to claim 1, wherein the delivered dose upon actuation of the metered dose inhaler contains the compound Mometasone Furoate in an amount of about 50 µg to about 400 µg.

4. The method according to claim 1, wherein the median particle size of the Mometasone Furoate that is delivered upon actuation of said metered dose inhaler remains less than about 5 microns.

5. The method according to claim 1, wherein the formulation has less than about 3 mg of non-volatile residue by weight per formulation.

6. The method according to claim 1, wherein the formulation has less than about 6 mg of valve extractables in the metering valve.

7. The method according to claim 1, wherein the metered dose inhaler further comprises a lubricant, wherein the formulation has less than about 100 µg of lubricant in the valve.

8. The method according to claim 7, wherein the formulation has less than about 50 µg of lubricant in the valve.

9. The method according to claim 1, wherein the delivered dose upon actuation of the metered dose inhaler contains the compound Formoterol in an amount of about 6 µg to about 12 µg.

10. The metered dose inhaler according to claim 8, wherein the particle size of the Formoterol that is delivered upon actuation of said metered dose inhaler remains less than about 5 microns.

11. The method according to claim 1, wherein the metered dose inhaler further comprises a surfactant.

12. The method according to claim 1, wherein the delivered dose upon actuation of the metered dose inhaler contains the compound Mometasone Furoate in an amount of about 50 µg to about 400 µg and wherein the delivered dose upon actuation of the metered dose inhaler contains the compound Formoterol in an amount of about 6 µg to about 12 µg.

13. The method according to claim 12, wherein the particle size of the Formoterol and the Mometasone Furoate that is delivered upon actuation of said metered dose inhaler remains less than about 5 microns.
